(19) Europäisches Patentamt / European Patent Office / Office européen des brevets

(11) **EP 4 446 308 A1**

(12) **EUROPEAN PATENT APPLICATION**
published in accordance with Art. 153(4) EPC

(43) Date of publication:
**16.10.2024 Bulletin 2024/42**

(21) Application number: **23865898.3**

(22) Date of filing: **15.09.2023**

(51) International Patent Classification (IPC):
***C07C 219/20*** *(2006.01)* ***A01N 33/12*** *(2006.01)*
***G01N 21/31*** *(2006.01)*

(52) Cooperative Patent Classification (CPC):
**A01N 33/12; A01P 1/00; C07C 219/08; C07C 219/20; G01N 21/31**

(86) International application number:
**PCT/KR2023/013917**

(87) International publication number:
**WO 2024/058602 (21.03.2024 Gazette 2024/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.09.2022 KR 20220117306**

(71) Applicant: **LG Chem, Ltd.**
**Yeongdeungpo-gu**
**Seoul 07336 (KR)**

(72) Inventors:
- **PARK, Saebomi**
  **Daejeon 34122 (KR)**
- **KANG, Yeonjoo**
  **Daejeon 34122 (KR)**
- **JANG, Bomee**
  **Daejeon 34122 (KR)**
- **HAN, Su Youn**
  **Daejeon 34122 (KR)**
- **KANG, Soonhee**
  **Daejeon 34122 (KR)**
- **LEE, Ji Seok**
  **Daejeon 34122 (KR)**
- **CHOI, Hyungsam**
  **Daejeon 34122 (KR)**

(74) Representative: **Plasseraud IP**
**104 Rue de Richelieu**
**CS92104**
**75080 Paris Cedex 02 (FR)**

(54) **COMPOUND AND METHOD FOR IDENTIFYING IMPURITIES INCLUDED THEREIN**

(57) The present invention relates to a compound and a method for confirming impurities included in the same.

[Figure 1]

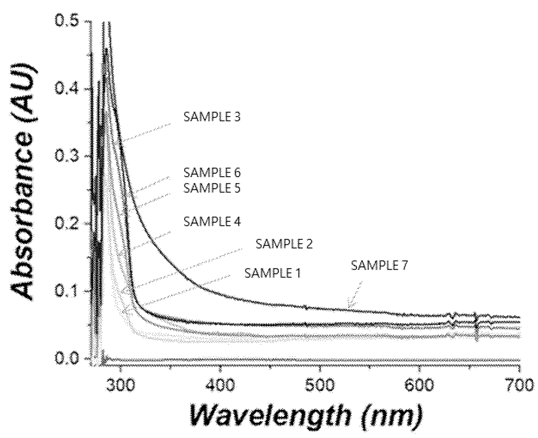


EP 4 446 308 A1

[Figure 1]

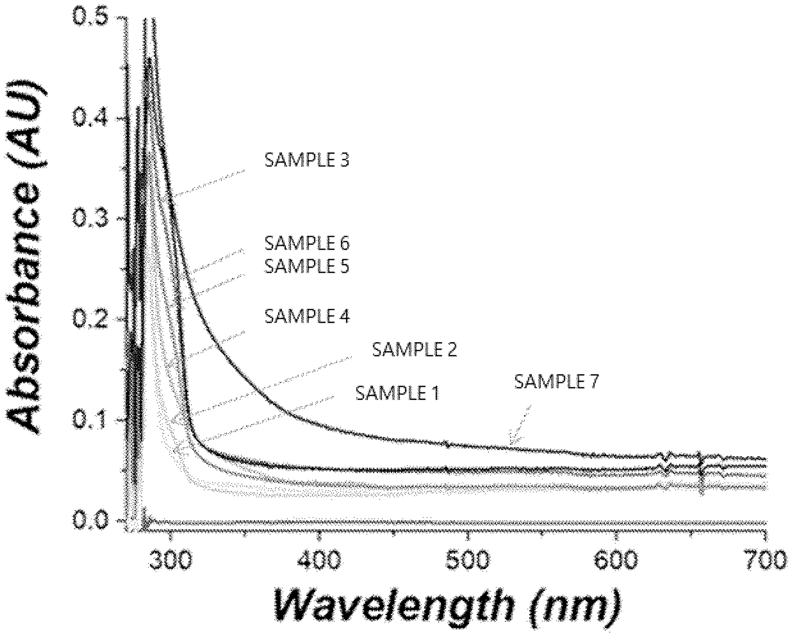
0.5
0.4
0.3
0.2
0.1
0.0
Absorbance (AU)
SAMPLE 3
SAMPLE 6
SAMPLE 5
SAMPLE 4
SAMPLE 2
SAMPLE 1
SAMPLE 7
300
400
500
600
700
Wavelength (nm)

## Description

[Technical Field]

This application claims priority to and the benefit of Korean Patent Application No. 10-2022-0117306 filed in the Korean Intellectual Property Office on September 16, 2022, the entire contents of which are incorporated herein by reference.
The present invention relates to a compound and a method for confirming impurities included in the same.

[Background Art]

Recently, various products such as daily supplies or hygiene products are required to have antibacterial properties.
The degree of antibacterial properties required and the material requirements for imparting antibacterial properties differ depending on the material of the product requiring antibacterial properties and the state of final use. For example, the properties of the material for imparting antibacterial properties and the degree of antibacterial properties vary depending on the amount of antibacterial material applied to a product used and the materials used together.
However, since products with the antibacterial properties described above include unintended decomposition products or intermediate products that are not a desired target due to a high temperature process required during the manufacturing process, it is difficult to obtain a highly pure product.
Therefore, there is a need for developing a method capable of confirming whether a product is an antibacterial material suitable for application to various products and includes decomposition products or impurities that are not a desired target.

[Detailed Description of the Invention]

[Technical Problem]

The present invention relates to a compound and a method for confirming impurities included in the same.

[Technical Solution]

An exemplary embodiment of the present invention provides a compound including a quaternary ammonium structure having an acrylate group or a methacrylate group, in which a turbidity derived from the following Method 1 is less than 120.

[Method 1]

A 20 wt% aqueous solution of the compound is analyzed by UV/Vis spectroscopy to measure the absorbance Abs to light at a wavelength of 290 nm, and the turbidity
Tb is derived from the measured absorbance Abs by the following Equation 1.

[Equation 1]

$$Abs = 0.00309\ Tb + 0.04946$$

Another exemplary embodiment of the present invention provides a method for confirming impurities in a compound including a quaternary ammonium structure having an acrylate group or a methacrylate group, in which the confirmation of impurities is performed by at least one of the following Methods 1' to 3'.

[Method 1']

A 20 wt% aqueous solution of the compound is analyzed by UV/Vis spectroscopy to measure the absorbance Abs to light at a wavelength of 290 nm, a turbidity Tb is derived from the measured absorbance Abs by the following Equation 1, and when the derived turbidity Tb is 120 or more, the presence of impurities is confirmed.

[Equation 1]

$$Abs = 0.00309\ Tb + 0.04946$$

[Method 2']

The compound is dissolved in a mixed solution including acetonitrile and water at a volume ratio of 5:5 and analyzed by HPLC/CAD, and when a peak of 100 mV or more is detected at 4 minutes to 9 minutes, the presence of impurities is confirmed.

[Method 3']

The compound is dissolved in DMF and analyzed by GPC/RI-DMF, and when a peak of 10 mV or more is detected at 10 minutes to 18 minutes, the presence of impurities is confirmed.

[Advantageous Effects]

The compound according to some exemplary embodiments of the invention has an advantage in that impurities are not included or scarcely included.

The compound according to some exemplary embodiments of the invention has an advantage of excellent antibacterial activity.

The method for confirming impurities according to some exemplary embodiments of the present invention can easily confirm whether a target impurity is included.

The method for confirming impurities according to some exemplary embodiments of the present invention can be applied to quality management such as quality control (QC).

The method for confirming impurities according to some exemplary embodiments of the present invention has an advantage of being performed in a short time.

[Brief Description of Drawings]

FIG. 1 is a view illustrating UV/Vis measurement results according to some exemplary embodiments of the present invention.

FIGS. 2 and 3 are views illustrating HPLC/CAD measurement results according to some exemplary embodiments of the present invention.

FIGS. 4 and 5 are views illustrating GPC/RI-DMF measurement results according to some exemplary embodiments of the present invention.

FIGS. 6 and 7 are views illustrating GPC/RI-$CHCl_3$ measurement results according to some exemplary embodiments of the present invention.

[Best Mode]

Hereinafter, the present invention will be described in detail.

An exemplary embodiment of the present invention provides a compound including a quaternary ammonium structure having an acrylate group or a methacrylate group, in which a turbidity derived from the following Method 1 is less than 120.

[Method 1]

A 20 wt% aqueous solution of the compound is analyzed by UV/Vis spectroscopy to measure the absorbance Abs to light at a wavelength of 290 nm, and a turbidity Tb is derived from the measured absorbance Abs by the following Equation 1.

[Equation 1]

$$Abs = 0.00309\ Tb + 0.04946$$

According to an exemplary embodiment of the present specification, Equation 1 is derived by the following Method A.

[Method A]

The absorbance of light at a wavelength of 290 nm is confirmed by analyzing 5 types of turbidity standard solutions (0, 1, 10, 100, and 1000 NTU, respectively) by UV/Vis spectroscopy (using Agilent 8453, 200 to 1100 nm, resolution 2 nm, 1 mm cuvette). In this case, an empty cuvette cell is used as a reference for measurement. A calibration curve (Equation 1) is derived from the absorbance according to the 0 NTU to 1,000 NTU interval. (NTU: Nephelometric Turbidity Unit, water quality standard 1 NTU)

According to an exemplary embodiment of the present invention, in Method 1, when the turbidity is 120 or more, it may be determined that a large amount of impurities are included, and when the turbidity is 40 or more and less than 120, it can be determined that some impurities are included. As an example, when the turbidity is 40 or more and less than 120, it can be determined that Sample Nos. 3 to 6 of the Examples to be described below include some impurities.

According to an exemplary embodiment of the present invention, the turbidity of the compound is less than 40. Preferably, when the turbidity Tb in Method 1 is less than 40, the absence of impurities can be confirmed. As an example, it can be determined that Sample Nos. 1 and 2 of the Examples to be described below, which have a turbidity Tb of less than 40, contain few or no impurities.

In contrast, when the turbidity Tb is 120 or more, it can be confirmed that the compound includes a large number of impurities. As an example, it can be determined that Sample No. 7 of the Examples to be described below, which has a turbidity Tb of 120 or more, includes a very large amount of impurities.

According to an exemplary embodiment of the present invention, the lower limit of the turbidity of the compound is not limited and may be 0 or more.

According to an exemplary embodiment of the present invention, when the turbidity of the compound satisfies a value less than 120, an effect of improving antibacterial activity may be exhibited.

According to an exemplary embodiment of the present invention, in order for the turbidity of the compound to satisfy the above range, that is, to reduce the concentration of impurities, the synthesis conditions of the compound may be adjusted, or purification may be performed during or after the synthesis process. Specifically, in order to reduce the concentration of impurities, the synthesis may be performed under a temperature condition of 20°C to 80°C for 3 hours to 30 hours during preparation of the compound. More specifically, the synthesis may be performed under a temperature condition of 40°C to 70°C, and may be performed for 4 hours to 25 hours. More specifically, the synthesis may be performed for 4 hours to 20 hours.

Furthermore, in order to reduce the concentration of impurities, the polymerization inhibitor may be used in an amount of 0.001 to 0.05 equivalents, specifically 0.001 to 0.01 equivalents, relative to 1 equivalent of the reactant during preparation of the compound.

In addition, in order to reduce the concentration of impurities, the ratio of a reaction solution and a precipitation solution during precipitation for the preparation of the compound may be a volume ratio of 1:1 to 1:30 (reaction solution: precipitation solution), specifically 1:3 to 1:20.

In an exemplary embodiment of the present invention, the polymerization inhibitor and the precipitation solution can be used without limitation as long as they are any materials used in the art.

According to an exemplary embodiment of the present invention, the impurity may be a polymer including a compound including a quaternary ammonium structure having an acrylate group or a methacrylate group and/or a polymer including a unit derived from a compound including a quaternary ammonium structure having an acrylate group or a methacrylate group. For example, the impurity may be a homopolymer of the compound.

According to an exemplary embodiment of the present invention, when the compound is subjected to the following Method 2, no peak of 100 mV or more is detected at 4 minutes to 9 minutes.

[Method 2]

The compound is dissolved in a mixed solution including acetonitrile and water at a volume ratio of 5:5 and analyzed using high performance liquid chromatography/charged aerosol detector (HPLC/CAD).

In this case, Method 2 may be performed under the following specific conditions.

- Column: Capcellpak CN, 4.6 mm ID × 150 mm L, 5um, Shiseido
- First mobile phase: acetonitrile/trichloroacetic acid = 100/0.1 (v/v, %)
- Second mobile phase: $H_2O$/trichloroacetic acid = 100/0.1 (v/v, %)
- Flow rate: 1 mL/min
- Injection amount: 5 μL
- Duration: 15 minutes

According to an exemplary embodiment of the present invention, a sample in which 1 mg of the compound is dissolved in 1 mL of the mixed solution may be used in Method 2. That is, the Method 2 is measured with a sample of concentration of 1 mg/mL (compound/mixed solution).

According to an exemplary embodiment of the present invention, a compound including a quaternary ammonium structure having an acrylate group or a methacrylate group, which does not include any impurities, does not have a peak of 100 mV or more at 4 minutes to 9 minutes when subjected to Method 2. In contrast, when impurities are mixed, the compound has a peak of 100 mV or more at 4 minutes to 9 minutes when subjected to Method 2. Based on this, it can be confirmed by Method 2 that when a peak of 100 mV or more is detected at 4 minutes to 9 minutes, a sample further includes impurities in addition to the compound including a quaternary ammonium structure having an acrylate group or a methacrylate group.

According to an exemplary embodiment of the present invention, when impurities are present, a peak of 125 mV or more is detected at 4 minutes to 9 minutes by Method 2 when the compound is subjected to Method 2.

According to an exemplary embodiment of the present invention, when the compound is subjected to Method 2, no peak of 50 mV or more is detected at 4 minutes to 9 minutes.

According to an exemplary embodiment of the present invention, when the compound is subjected to Method 2, no peak or a peak of less than 50 mV is detected at 4 minutes to 9 minutes.

According to an exemplary embodiment of the present invention, when the compound is subjected to Method 2, no peak of 100 mV or more is detected at 4 minutes to 12 minutes.

According to an exemplary embodiment of the present invention, when the compound is subjected to Method 2, a peak is detected at 1 minute to 3 minutes.

According to an exemplary embodiment of the present invention, when the compound is subjected to Method 2, a peak of 100 mV or more is detected at 1 minute to 3 minutes.

According to an exemplary embodiment of the present invention, when the compound is subjected to Method 2, a peak is detected at 1 minute to 3 minutes, and one or more of the detected peaks are 100 mV to 5,000 mV.

In the present invention, the size of a peak means the size of the highest point among the peaks. For example, the detection of a peak of 100 mV or more at 1 minute to 3 minutes means that the highest point of the peak present between 1 minute to 3 minutes is 100 mV or more.

According to an exemplary embodiment of the present invention, when Method 2 is used, compounds from which no peak of 100 mV or more is detected at 4 minutes to 9 minutes exhibit an effect of improving antibacterial activity.

According to an exemplary embodiment of the present invention, when Method 2 is used, in order to prevent peaks of 100 mV or more from being detected at 4 minutes to 9 minutes, that is, to reduce the concentration of impurities, the synthesis conditions of the compound may be adjusted or purification may be performed during or after the synthesis process. The specific synthesis conditions for this are the same as those described above in Method 1.

According to an exemplary embodiment of the present invention, when the compound is subjected to the following Method 3, no peak of 10 mV or more is detected at 10 minutes to 18 minutes.

[Method 3]

The compound is dissolved in DMF and analyzed by gel permeation chromatography/refractive index-DMF (GPC/RI-DMF).

In this case, Method 3 may be performed under the following specific conditions.

- Column: PLgel column
- First mobile phase: DMF/0.01M LiBr
- Flow rate: 1 mL/min
- Injection amount: 100 μL
- Duration: 35 minutes

According to an exemplary embodiment of the present invention, a sample in which 5 mg of the compound is dissolved in 1 mL of dimethylformamide (DMF) may be used in Method 3. That is, the Method 3 is measured with a sample of concentration of 5 mg/mL (compound/mixed solution).

According to an exemplary embodiment of the present invention, a compound including a quaternary ammonium structure having an acrylate group or a methacrylate group, which does not include any impurities, does not have a peak of 10 mV or more at 10 minutes to 18 minutes when subjected to Method 3. In contrast, when impurities are mixed, the compound has a peak of 10 mV or more at 10 minutes to 18 minutes when subjected to Method 3. Based on this, it can be confirmed by Method 3 that when a peak of 10 mV or more is detected at 10 minutes to 18 minutes, a sample further includes impurities in addition to the compound including a quaternary ammonium structure having an acrylate group or a methacrylate group.

According to an exemplary embodiment of the present invention, when the compound is subjected to Method 3, no peak of 5 mV or more is detected at 10 minutes to 18 minutes.

According to an exemplary embodiment of the present invention, when the compound is subjected to Method 3, no peak is detected at 10 minutes to 18 minutes.

According to an exemplary embodiment of the present invention, when a sample including impurities is subjected to Method 3, a peak of 10 mV or more is detected at 10 minutes to 18 minutes.

According to an exemplary embodiment of the present invention, the upper limit of a peak detected from a sample including impurities is not limited, but may be, for example, 500 mV or less. For example, when a sample including impurities is subjected to Method 3, a peak of 10 mV or more and 500 mV or less is detected at 10 minutes to 18 minutes.

According to an exemplary embodiment of the present invention, in Method 3, $CHCl_3$ is dissolved in DMF at a concentration of 5 mg/mL (sample/DMF) and mixed (shaken) for about 10 hours to 14 hours, and then GPC/RI-DMF may be measured.

According to an exemplary embodiment of the present invention, when Method 3 is used, compounds from which no peak of 10mV or more is detected at 10 minutes to 18 minutes exhibit an effect of improving antibacterial activity.

According to an exemplary embodiment of the present invention, when Method 3 is used, in order to prevent peaks of 10mV or more from being detected at 10 minutes to 18 minutes, that is, to reduce the concentration of impurities, the synthesis conditions of the compound may be adjusted or purification may be performed during or after the synthesis process. The specific synthesis conditions for this are the same as those described above in Method 1.

According to an exemplary embodiment of the present invention, the compound is represented by the following Chemical Formula 1.

[Chemical Formula 1]

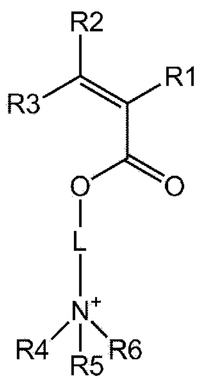

In Chemical Formula 1,

R1 to R3 are the same as or different from each other, and are each independently hydrogen or a methyl group,
R4 to R6 are the same as or different from each other, and are each independently an alkyl group, and
L is an alkylene group.

According to an exemplary embodiment of the present invention, R1 is hydrogen or a methyl group.

According to an exemplary embodiment of the present invention, R2 is hydrogen or a methyl group.

According to an exemplary embodiment of the present invention, R3 is hydrogen or a methyl group.

According to an exemplary embodiment of the present invention, R2 and R3 are hydrogen, and R1 is hydrogen or a methyl group.

According to an exemplary embodiment of the present invention, L is an alkylene group having 1 to 30 carbon atoms.

According to an exemplary embodiment of the present invention, L is an alkylene group having 1 to 10 carbon

atoms.

According to an exemplary embodiment of the present invention, L is an alkylene group having 1 to 5 carbon atoms.

According to an exemplary embodiment of the present invention, L is a methylene group, an ethylene group, a propylene group or a butylene group.

According to an exemplary embodiment of the present invention, R2 and R3 are hydrogen, R1 is hydrogen or a methyl group, and L is an alkylene group having 1 to 30 carbon atoms.

According to an exemplary embodiment of the present invention, R2 and R3 are hydrogen, R1 is hydrogen or a methyl group, and L is an alkylene group having 1 to 10 carbon atoms.

According to an exemplary embodiment of the present invention, R2 and R3 are hydrogen, R1 is hydrogen or a methyl group, and L is a methylene group, an ethylene group, a propylene group or a butylene group.

According to an exemplary embodiment of the present invention, R4 to R6 are the same as or different from each other, and are each independently an alkyl group having 1 to 30 carbon atoms.

According to an exemplary embodiment of the present invention, any one of R4 to R6 is an alkyl group having 5 to 30 carbon atoms, and the others are the same as or different from each other, and are each independently an alkyl group having 1 to 30 carbon atoms.

According to an exemplary embodiment of the present invention, any one of R4 to R6 is an alkyl group having 5 to 30 carbon atoms, and the others are the same as or different from each other, and are each independently an alkyl group having 1 to 20 carbon atoms.

According to an exemplary embodiment of the present invention, any one of R4 to R6 is an alkyl group having 5 to 30 carbon atoms, and the others are the same as or different from each other, and are each independently an alkyl group having 1 to 15 carbon atoms.

According to an exemplary embodiment of the present invention, any one of R4 to R6 is an alkyl group having 5 to 20 carbon atoms, and the others are the same as or different from each other, and are each independently an alkyl group having 1 to 30 carbon atoms.

According to an exemplary embodiment of the present invention, any one of R4 to R6 is an alkyl group having 5 to 20 carbon atoms, and the others are the same as or different from each other, and are each independently an alkyl group having 1 to 20 carbon atoms.

According to an exemplary embodiment of the present invention, any one of R4 to R6 is an alkyl group having 5 to 20 carbon atoms, and the others are the same as or different from each other, and are each independently an alkyl group having 1 to 10 carbon atoms.

According to an exemplary embodiment of the present invention, any one of R4 to R6 is an alkyl group having 5 to 20 carbon atoms, and the others are the same as or different from each other, and are each independently an alkyl group having 1 to 4 carbon atoms.

According to an exemplary embodiment of the present invention, R2 and R3 are hydrogen, R1 is hydrogen or a methyl group, L is an alkylene group having 1 to 30 carbon atoms, any one of R4 to R6 is an alkyl group having 5 to 30 carbon atoms, and the others of R4 to R6 are the same as or different from each other, and are each independently an alkyl group having 1 to 30 carbon atoms.

According to an exemplary embodiment of the present invention, R2 and R3 are hydrogen, R1 is hydrogen or a methyl group, L is an alkylene group having 1 to 20 carbon atoms, any one of R4 to R6 is an alkyl group having 5 to 20 carbon atoms, and the others of R4 to R6 are the same as or different from each other, and are each independently an alkyl group having 1 to 20 carbon atoms.

According to an exemplary embodiment of the present invention, R2 and R3 are hydrogen, R1 is hydrogen or a methyl group, L is an alkylene group having 1 to 10 carbon atoms, any one of R4 to R6 is an alkyl group having 5 to 20 carbon atoms, and the others of R4 to R6 are the same as or different from each other, and are each independently an alkyl group having 1 to 15 carbon atoms.

According to an exemplary embodiment of the present invention, the compound is any one selected from the following Compounds 1 to 10.

Compound 1

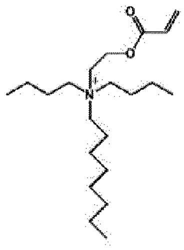

Compound 2

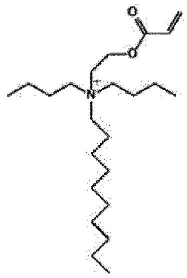

Compound 3

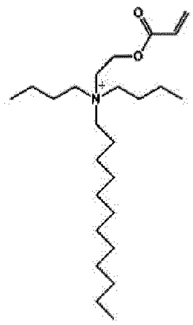

Compound 4

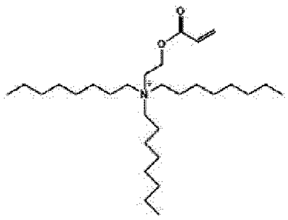

Compound 5

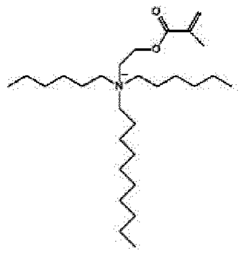

Compound 6

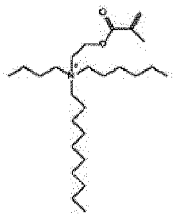

Compound 7

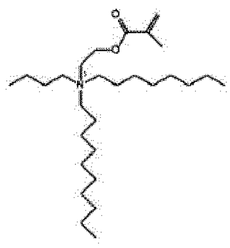

Compound 8

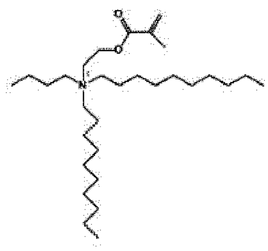

Compound 9

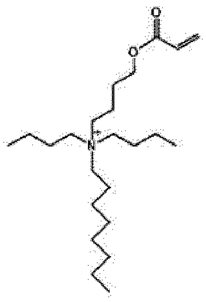

Compound 10

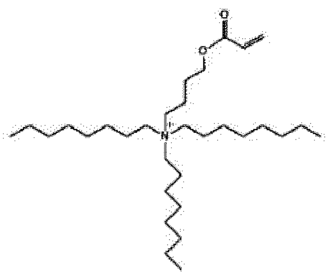

According to an exemplary embodiment of the present invention, since the compound including the quaternary ammonium structure having an acrylate group or methacrylate group exhibits cationic properties, the compound may be present in a form where a salt is formed along with a group exhibiting anionic properties. In this case, the group exhibiting anionic properties is not particularly limited, and materials known in the art may be used as long as the materials do not impair the purpose of the antibacterial resin. For example, the group exhibiting anionic properties may be a halogen-based anion or a sulfonate-based anion, and specifically may be Br-, but is not limited thereto.

According to an exemplary embodiment of the present invention, the compound is an antibacterial compound. That is, the compound exhibits antibacterial properties.

In an exemplary embodiment of the present specification, the antibacterial resin exhibits antibacterial properties

against at least one of Gram-positive bacteria, Gram-negative bacteria, and molds.

As used herein, the term Gram-positive bacteria is a general term for bacteria that are stained purple when stained using the Gram staining method, and Gram-positive bacteria exhibit a purple color without discoloration even though the Gram-positive bacteria are stained with a basic dye such as crystal violet and then treated with ethanol because the cell walls of Gram-positive bacteria are composed of several layers of peptidoglycan.

In an exemplary embodiment of the present specification, the Gram-positive bacteria are selected from *Enterococcus faecalis, Staphylococcus aureus, Streptococcus pneumoniae, Enterococcus faecium* and *Lactobacillus lactis.* Specifically, the Gram-positive bacteria are any one selected from the above-described examples, but are not limited thereto.

As used herein, the term Gram-negative bacteria is a general term for bacteria that are stained red when stained with the Gram staining method, and Gram-negative bacteria have an outer membrane composed of lipid polysaccharides, lipid proteins, and/or other complex polymeric materials instead of having a cell wall with a relatively small amount of peptidoglycan compared to Gram-positive bacteria.

In an exemplary embodiment of the present specification, the Gram-negative bacteria are selected from Proteus mirabilis, Escherichia coli, Salmonella typhi, Pseudomonas aeruginosa and Vibrio cholerae. Specifically, the Gram-negative bacteria are any one selected from the above-described examples, but are not limited thereto.

In an exemplary embodiment of the present specification, the mold may be Candida albicans, but is not limited thereto.

In the present specification, exhibiting antibacterial properties means that the antibacterial activity measured based on the following Method 4, in other words, the bacteriostatic reduction rate is 90% or more.

According to an exemplary embodiment of the present specification, the antibacterial resin has an antibacterial activity of 90% or more against any one bacterial strain selected from the group consisting of Gram-positive bacteria, Gram-negative bacteria, and molds, as measured by the following Method 4.

[Method 4]

After 1 g of a sample was put into a 250 mL Erlenmeyer flask, 50 ml of phosphate buffered saline (PBS) inoculated with a bacterial strain was injected. The sample was cultured in a shaking incubator maintained at 35°C for 1 hour. The culture solution was diluted and smeared on an agar medium plate. Serial dilution was performed to enable colony counting, and a 0.9 wt.% NaCl solution was used in this process. After the microbial concentration ($C_0$, CFU/ml) at the initial concentration was calculated in consideration of the dilution concentration, an antibacterial activity was derived by calculating the following Mathematical Equation 1.

[Mathematical Equation 1]

$$\text{Antibacterial activity}(\%) = \left(1 - A_{Sample}/A_{Reference}\right) \times 100$$

$A_{Sample}$ = Concentration of microorganisms in medium incubated by adding sample

$A_{Reference}$ = Concentration of microorganisms in medium incubated without adding sample

In an exemplary embodiment of the present specification, the sample used in Method 4 is a sample including the compound.

In an exemplary embodiment of the present specification, the *E. coli* ATCC 25922 standard strain is used as the bacterial strain when Method 4 is performed.

In an exemplary embodiment of the present specification, when Method 4 is performed, the strain is inoculated at $1.5\times10^5$ CFU/ml to $3\times10^5$ CFU/ml and used.

In an exemplary embodiment of the present specification, the antibacterial resin has an antibacterial activity of 90% or more against Gram-positive bacteria as measured by Method 4.

In an exemplary embodiment of the present specification, the compound has an antibacterial activity of 90% or more against Gram-negative bacteria as measured by Method 4.

In an exemplary embodiment of the present specification, the compound has an antibacterial activity of 90% or more against molds as measured by Method 4.

According to an exemplary embodiment of the present specification, the antibacterial activity is 92% or more, 94% or more, 96% or more, 98% or more, 99% or more or 99.8% or more. The upper limit of the antibacterial activity is not particularly limited, and is, for example, 100% or less or less than 100%.

Since the bacterial strains of the Gram-positive bacteria, Gram-negative bacteria and molds may not only induce various diseases upon contact, but also cause secondary infections, it is preferred to exhibit antibacterial properties

against all of the Gram-positive bacteria, Gram-negative bacteria, and molds using one compound.

An exemplary embodiment of the present invention provides a method for confirming impurities as described below. Specifically, the method is a method for confirming impurities in a compound including a quaternary ammonium structure having an acrylate group or a methacrylate group, in which the confirmation of impurities is performed by at least one of the following Methods 1' to 3'.

[Method 1']

A 20 wt% aqueous solution of the compound is analyzed by UV/Vis spectroscopy to measure the absorbance Abs to light at a wavelength of 290 nm, a turbidity Tb is derived from the measured absorbance Abs by the following Equation 1, and when the derived turbidity Tb is 120 or more, the presence of impurities is confirmed.

[Equation 1]

$$Abs = 0.00309\ Tb + 0.04946$$

[Method 2']

The compound is dissolved in a mixed solution including acetonitrile and water at a volume ratio of 5:5 and analyzed by HPLC/CAD, and when a peak of 100 mV or more is detected at 4 minutes to 9 minutes, the presence of impurities is confirmed.

[Method 3']

The compound is dissolved in DMF and analyzed by GPC/RI-DMF, and when a peak of 10 mV or more is detected at 10 minutes to 18 minutes, the presence of impurities is confirmed.

In this case, Method 2' may be performed under the following specific conditions.

- Column: Capcellpak CN, 4.6 mm ID × 150 mm L, 5 um, Shiseido
- First mobile phase: acetonitrile/trichloroacetic acid = 100/0.1 (v/v, %)
- Second mobile phase: $H_2O$/trichloroacetic acid = 100/0.1 (v/v, %)
- Flow rate: 1 mL/min
- Injection amount: 5 μL
- Duration: 15 minutes

In this case, Method 3' may be performed under the following specific conditions.

- Column: PLgel column
- First mobile phase: DMF/0.01M LiBr
- Flow rate: 1 mL/min
- Injection amount: 100 μL
- Duration: 35 minutes

According to an exemplary embodiment of the present invention, the impurity may be a polymer including a compound including a quaternary ammonium structure having an acrylate group or a methacrylate group and/or a polymer including a unit derived from a compound including a quaternary ammonium structure having an acrylate group or a methacrylate group. For example, the impurity may be a homopolymer of the compound.

In general, the compound including a quaternary ammonium structure having an acrylate group or a methacrylate group has extremely high solubility to water, an organic solvent, and the like, so that the structure thereof can be easily confirmed using a general solution 1H NMR spectrum. In contrast, a polymer including the compound including a quaternary ammonium structure having an acrylate group or a methacrylate group and a polymer including a unit derived from the compound including a quaternary ammonium structure having an acrylate group or a methacrylate group has low solubility to water, an organic solvent, and the like, so that it is difficult to confirm the structure using the solution NMR spectrum. That is, in a state in which a compound including a quaternary ammonium structure having an acrylate group or a methacrylate group is mixed with impurities, it is difficult to confirm whether impurities are included using the solution NMR spectrum.

The method for confirming impurities according to the present invention has an advantage in that impurities

can be easily confirmed even in a state in which the impurities and the compound including a quaternary ammonium structure having an acrylate group or a methacrylate group are mixed.

Furthermore, it takes a long experimental time for the method such as solid NMR to be performed, whereas it does not take a long time for the method for confirming impurities of the present invention to be performed. Accordingly, the method for confirming impurities of the present invention has an advantage of being applicable to quality management such as quality control (QC).

As an example, homopolymer impurities may be generated in the scale-up synthesis process of the compound, and the grade according to the amount of impurities may be easily and quickly evaluated through the impurity confirmation method described above.

According to an exemplary embodiment of the present invention, the description on the above-described Method 1 can be applied to Method 1’ used in the impurity confirmation method, the description on the above-described Method 2 can be applied to Method 2’, and the description on the above-described Method 3 can be applied to Method 3’.

An exemplary embodiment of the present invention provides a composition including the above-described compound.

According to an exemplary embodiment of the present invention, the composition is an antibacterial composition.

According to an exemplary embodiment of the present invention, the composition may be composed of the above-described compounds. For example, the composition may be composed of compounds having a turbidity of less than 120 derived from Method 1, as a compound including a quaternary ammonium structure having an acrylate group or a methacrylate group.

According to an exemplary embodiment of the present invention, the composition may include the above-described compound and the above-described impurities. For example, the composition may be composed of compounds having a turbidity of less than 120 and compounds having a turbidity of 120 or more derived from Method 1, as compounds including a quaternary ammonium structure having an acrylate group or a methacrylate group.

According to an exemplary embodiment of the present invention, the composition may further include an additive in addition to the compound.

According to an exemplary embodiment of the present invention, the additive may be an additive exhibiting an antibacterial effect.

According to an exemplary embodiment of the present invention, the additive can be applied without limitation as long as it is a material used in the art.

When one member (layer) is disposed "on" another member (layer) in the present invention, this includes not only a case where the one member (layer) is brought into contact with another member (layer), but also a case where still another member (layer) is present between the two members (layers).

When one part "includes" one constituent element in the present invention, unless otherwise specifically described, this does not mean that another constituent element is excluded, but means that another constituent element may be further included.

[Mode for Invention]

Hereinafter, the present invention will be described in detail with reference to Examples for specifically describing the present invention. However, the Examples according to the present invention may be modified into various different forms, and it should not be interpreted that the scope of the present invention is limited to the Examples to be described below. The Examples of the present invention are provided for more completely explaining the present invention to the person with ordinary skill in the art.

**<Preparation Example A> Synthesis of compounds**

**A-1. Synthesis of Compound 1, Compound 2, and** Compound 3

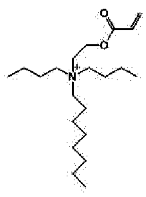

1

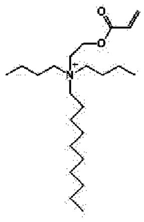

2

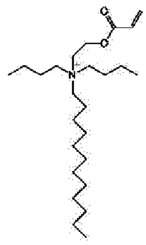

3

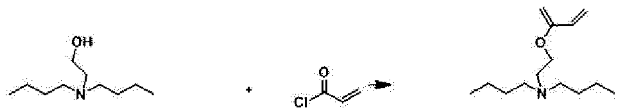

Step 1.

(1) 0.1 mol of 2-(dibutylamino)ethanol (DBAE), 0.1 mol of trimethylamine and 0.001 mol of hydroquinone were put into 100 mL of tetrahydrofuran (THF)(solvent).
(2) 0.1 mol of acryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromooctane (preparation of Compound 1), 1-bromodecane (preparation of Compound 2) or 1-bromododecane (preparation of Compound 3) were dissolved at 50 wt% in acrylonitrile (solvent) at a ratio of 1:1.
(2) Subsequently, p-methoxyphenol, which is a polymerization inhibitor, was added (ratio with reactants 1:0.001 eq).
(3) The resulting mixture was reacted at 50°C for 20 hours.
(4) After static precipitation (MTBE:reaction solution = 15:1) in methyl t-butyl ether (MTBE), the mixture was filtered.
(5) The resulting product was dried under vacuum at 45°C.

**A-2. Synthesis of Compound 4**

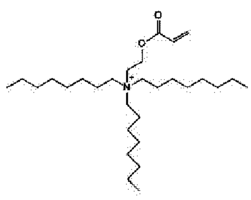

4

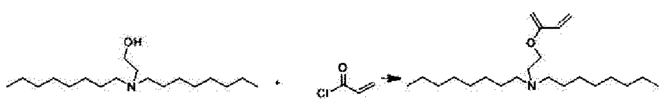

Step 1

(1) 0.1 mol of 2-(dioctylamino)ethanol (DOAE), 0.1 mol of trimethylamine and 0.001 mol of hydroquinone were put into 100 mL of THF (solvent).
(2) 0.1 mol of acryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2

(1) The product of Step 1 and 1-bromooctane were dissolved at 50 wt% in acrylonitrile (solvent) at a ratio of 1:1.
(2) Subsequently, p-methoxyphenol, which is a polymerization inhibitor, was added (ratio with reactants 1:0.001 eq).
(3) The resulting mixture was reacted at 50°C for 20 hours.
(4) After static precipitation (MTBE:reaction solution = 15:1) in methyl t-butyl ether (MTBE), the mixture was filtered.
(5) The resulting product was dried under vacuum at 45°C.

**A-3. Synthesis of Compound 5**

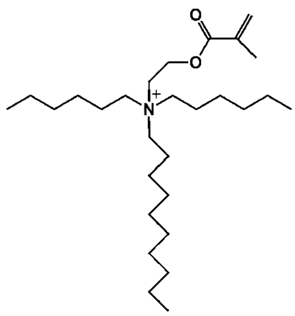

5

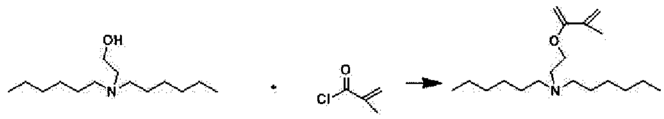

Step 1.

(1) 0.1 mol of 2-(dihexylamino)ethanol (DHAE), 0.1 mol of trimethylamine and 0.001 mol of hydroquinone were put into 100 mL of THF (solvent).
(2) 0.1 mol of methacryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromodecane were dissolved at 50 wt% in acrylonitrile (solvent) at a ratio of 1:1.
(2) Subsequently, p-methoxyphenol, which is a polymerization inhibitor, was added (ratio with reactants 1:0.001 eq).
(3) The resulting mixture was reacted at 50°C for 20 hours.
(4) After static precipitation (MTBE:reaction solution = 15:1) in methyl t-butyl ether (MTBE), the mixture was filtered.
(5) The resulting product was dried under vacuum at 45°C.

**A-4. Synthesis of Compound 6**

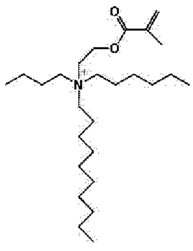

6

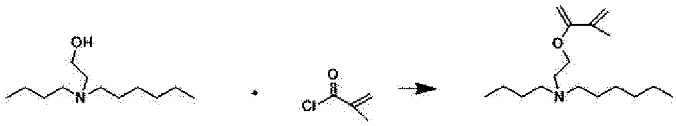

Step 1.

(1) 0.1 mol of 2-(butylhexylamino)ethanol (BHAE), 0.1 mol of trimethylamine and 0.001 mol of hydroquinone were put into 100 mL of THF (solvent).
(2) 0.1 mol of methacryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromodecane were dissolved at 50 wt% in acrylonitrile (solvent) at a ratio of 1:1.
(2) Subsequently, p-methoxyphenol, which is a polymerization inhibitor, was added (ratio with reactants 1:0.001 eq).
(3) The resulting mixture was reacted at 50°C for 20 hours.
(4) After static precipitation (MTBE:reaction solution = 15:1) in methyl t-butyl ether (MTBE), the mixture was filtered.
(5) The resulting product was dried under vacuum at 45°C.

**A-5. Synthesis of Compound 7**

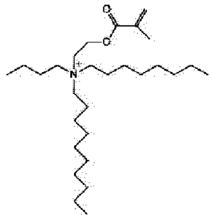

7

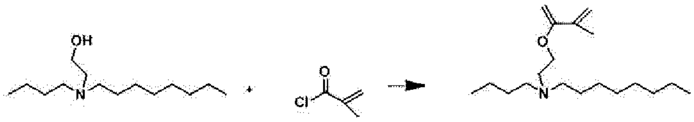

Step 1.

(1) 0.1 mol of 2-(butyloctylamino)ethanol (BOAE), 0.1 mol of trimethylamine and 0.001 mol of hydroquinone were put into 100 mL of THF (solvent).
(2) 0.1 mol of methacryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromodecane were dissolved at 50 wt% in acrylonitrile (solvent) at a ratio of 1:1.
(2) Subsequently, p-methoxyphenol, which is a polymerization inhibitor, was added (ratio with reactants 1:0.001 eq).
(3) The resulting mixture was reacted at 50°C for 20 hours.
(4) After static precipitation (MTBE:reaction solution = 15:1) in methyl t-butyl ether (MTBE), the mixture was filtered.
(5) The resulting product was dried under vacuum at 45°C.

**A-6. Synthesis of Compound 8**

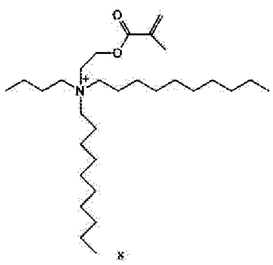

8

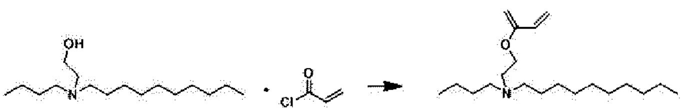

Step 1.

(1) 0.1 mol of 2-(butyldecylamino)ethanol (BDAE), 0.1 mol of trimethylamine and 0.001 mol of hydroquinone were put into 100 mL of THF (solvent).
(2) 0.1 mol of methacryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromodecane were dissolved at 50 wt% in acrylonitrile (solvent) at a ratio of 1:1.
(2) Subsequently, p-methoxyphenol, which is a polymerization inhibitor, was added (ratio with reactants 1:0.001 eq).
(3) The resulting mixture was reacted at 50°C for 20 hours.
(4) After static precipitation (MTBE:reaction solution = 15:1) in methyl t-butyl ether (MTBE), the mixture was filtered.
(5) The resulting product was dried under vacuum at 45°C.

**A-7. Synthesis of Compound 9**

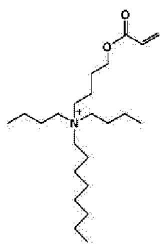

9

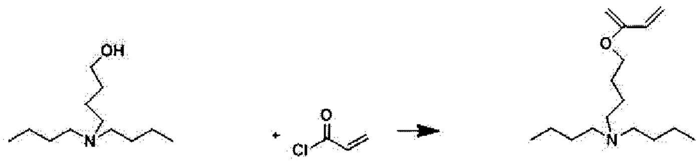

Step 1.

(1) 0.1 mol of 2-(dibutylamino)butanol (DBAB), 0.1 mol of trimethylamine and 0.001 mol of hydroquinone were put into 100 mL of THF (solvent).
(2) 0.1 mol of acryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromooctane were dissolved at 50 wt% in acrylonitrile (solvent) at a ratio of 1:1.
(2) Subsequently, p-methoxyphenol, which is a polymerization inhibitor, was added (ratio with reactants 1:0.001 eq).
(3) The resulting mixture was reacted at 50°C for 20 hours.

(4) After static precipitation (MTBE:reaction solution = 15:1) in methyl t-butyl ether (MTBE), the mixture was filtered.
(5) The resulting product was dried under vacuum at 45°C.

### A-8. Synthesis of Compound 10

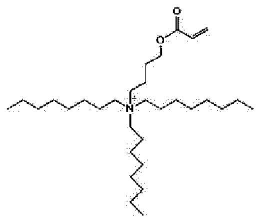

10

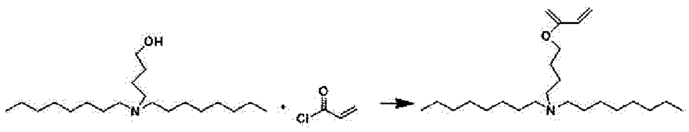

Step 1.

(1) 0.1 mol of 2-(dioctylamino)butanol (DOAB), 0.1 mol of trimethylamine and 0.001 mol of hydroquinone were put into 100 mL of THF (solvent).
(2) 0.1 mol of acryloyl chloride was added dropwise onto a reaction solution while stirring the materials (room temperature).
(3) The resulting mixture was stirred for 2 hours.
(4) After a triethylamine salt was removed by filtering the mixture, the solvent was removed by a rotary evaporator.
(5) The residue was dried under vacuum at 83°C to 87°C.

Step 2.

(1) The product of Step 1 and 1-bromooctane were dissolved at 50 wt% in acrylonitrile (solvent) at a ratio of 1:1.
(2) Subsequently, p-methoxyphenol, which is a polymerization inhibitor, was added (ratio with reactants 1:0.001 eq).
(3) The resulting mixture was reacted at 50°C for 20 hours.
(4) After static precipitation (MTBE:reaction solution = 15:1) in methyl t-butyl ether (MTBE), the mixture was filtered.
(5) The resulting product was dried under vacuum at 45°C.

Although a static precipitation method of adding a reactant to a nonsolvent was used in the above-described Synthesis Examples, a reverse precipitation method of adding a nonsolvent to a reactant may also be used. Furthermore, in addition to 15:1, other ratios of MTBE and reaction solution may be used, and for example, 12:1 and 26:1 may be used.

### <Preparation Example B> Preparation of sample

During the preparation of Compound 3 in step 2(3) of Preparation Example A-1 above, the products were collected and named Samples 1 to 7 when the polymerization was performed for a reaction time of 4 hours, 8 hours, 10 hours, 12 hours, 16 hours, 20 hours, and 24 hours, respectively.

**<Examples>**

Example 1. Measurement of turbidity

UV/Vis was measured for Samples 1 to 7 prepared in Preparation Example B above using Method 1 of the present invention, and the results are illustrated in FIG. 1. Further, values obtained by converting the measured absorbance (Abs) into turbidity using the following Equation 1 are shown in the following Table 1.

[Equation 1]

$$Abs = 0.00309\ Tb + 0.04946$$

Specifically, UV/Visspectroscopy was analyzed and turbidity was measured under the following conditions.

1) Measurement of UV/Vis spectroscopy

500 mg of each of Samples 1 to 7 were put into a vial, a 20 wt% aqueous solution was prepared using distilled water (DI water), and then UV/Vis spectroscopy (using Agilent 8453, 200 to 1100nm, resolution 2 nm, 1 mm cuvette) was analyzed to confirm the absorbance of light at a wavelength of 290 nm. In this case, DI water was used as a reference for measurement.

2) Derivation of turbidity calculation equation

(Equation 1)

The absorbance of light at a wavelength of 290 nm was confirmed by analyzing 5 types of turbidity standard solutions (0, 1, 10, 100, and 1000 NTU, respectively) by UV/Vis spectroscopy (using Agilent 8453, 200 to 1100 nm, resolution 2 nm, 1 mm cuvette). In this case, an empty cuvette cell was used as a reference for measurement.

A calibration curve (Equation 1) was derived from the absorbance according to the 0 NTU to 1,000 NTU interval. (NTU: Nephelometric Turbidity Unit, water quality standard 1 NTU)

[Equation 1]

$$Abs = 0.00309\ Tb + 0.04946$$

(Abs: absorbance, Tb: turbidity)

3) Calculation of turbidity of Samples 1 to 7

Turbidity was derived by introducing the absorbance of Samples 1 to 7 confirmed in 1) above into Equation 1.

[Table 1]

| Sample No. | Absorbance | Turbidity |
|---|---|---|
| 1 | 0.1428 | 30 |
| 2 | 0.1615 | 36 |
| 3 | 0.3459 | 96 |
| 4 | 0.2301 | 58 |
| 5 | 0.2796 | 74 |
| 6 | 0.3967 | 112 |
| 7 | 0.4568 | 132 |

From Table 1 above, it can be confirmed that as the polymerization progresses and the amount of homopolymer

relative to the compound increases (from Samples 1 to 7), the turbidity is improved. Specifically, it can be confirmed that Samples 1 and 2, in which a small amount of homopolymer was produced due to a short polymerization time, had a converted turbidity of less than 40. In addition, it can be confirmed that Samples 3 to 6, in which the polymerization time was intermediate, produced a small amount of homopolymer, and thus had a turbidity of 40 or more and less than 120. In contrast, in the case of Sample 7 in which a large amount of homopolymer was produced due to a long polymerization time, it can be confirmed that the turbidity was 120 or more.

From this, it can be seen that the degree of impurities included may be confirmed through turbidity measurement using Method 1 and Equation 1.

Example 2. Measurement of HPLC/CAD

Impurities in Sample 1 and Sample 6 were confirmed using Method 2 of the present invention.

Specifically, HPLC/CAD was measured under the following conditions.

- Column: Capcellpak CN, 4.6 mm ID × 150 mm L, 5 um, Shiseido
- First mobile phase: acetonitrile/trichloroacetic acid = 100/0.1 (v/v, %)
- Second mobile phase: $H_2O$/trichloroacetic acid = 100/0.1 (v/v, %)
- Flow rate: 1 mL/min
- Injection amount: 5 μL
- Duration: 15 minutes

FIG. 2 illustrates the HPLC/CAD measurement results of Sample 1. Since no impurities other than Compound 3 were included during the measurement in FIG. 2, it can be confirmed that two or more peaks are derived only at 1 minute to 3 minutes, and no peaks are derived at 4 minutes to 9 minutes.

FIG. 3 illustrates the HPLC/CAD measurement results of Sample 6. Since Sample 6 used in FIG. 3 includes Compound 3 and impurities, it can be confirmed that a peak of 100 mV or more was detected at 4 minutes to 9 minutes along with two or more peaks detected at 1 minute to 3 minutes.

Example 3. Measurement of GPC/RI-DMF

Impurities in Sample 1 and Sample 7 were confirmed using Method 3 of the present invention.

Specifically, GPC/RI-DMF was measured under the following conditions.

- Column: PLgel column
- First mobile phase: DMF/0.01M LiBr
- Flow rate: 1 mL/min
- Injection amount: 100 μL
- Duration: 35 minutes

FIG. 4 illustrates the GPC/RI-DMF measurement results of Sample 1, and FIG. 5 illustrates the GPC/RI-DMF measurement results of Sample 7.

FIG. 4 illustrates the measurement using Sample 1, which contains a compound and a trace amount of homopolymer, FIG. 5 illustrates the measurement using Sample 7, which contains a compound and a large amount of homopolymer, and it can be confirmed that a peak of 10 mV or more is detected at 10 minutes to 18 minutes in both FIGS. 4 and 5. Through this, it was confirmed that even though only a trace amount of homopolymer (impurity) is included in a compound, it can be detected through GPC/RI-DMF measurement as in Method 3 whether impurities are included.

Comparative Example 1. Measurement of GPC/RI-$CHCl_3$

In Example 3 above, GPC/RI-$CHCl_3$ was measured for Sample 1 and Sample 6 using $CHCl_3$ instead of DMF.

FIG. 6 illustrates the measurement results of Compound 3 prepared in Preparation Example A-1, and FIG. 7 illustrates the GPC/RI-$CHCl_3$ measurement results of Sample 6.

From FIG. 6, it can be confirmed that for the compound of the present invention, a peak of 20 mV or more was detected at 18 minutes to 22 minutes during the measurement of GPC/RI-$CHCl_3$. In contrast, it can be confirmed in FIG. 7 that no peaks were detected at 18 minutes to 22 minutes. Sample 6 includes the compound and impurities, and a peak of 20 mV or more needs to be detected at 18 minutes to 22 minutes as illustrated in FIG. 6 even in FIG. 7, but such a peak was not detected in FIG. 7, so that it was confirmed that GPC/RI-$CHCl_3$ is not suitable for the detection of impurities.

Example 4. Measurement of antibacterial activity

After 1 g of a sample was put into a 250 mL Erlenmeyer flask, 50 ml of phosphate buffered saline (PBS) inoculated with *E. coli* ATCC 25922 standard bacterial strain was injected. The sample was cultured in a shaking incubator maintained at 35°C for 1 hour. The culture solution was diluted and smeared on an agar medium plate. Serial dilution was performed to enable colony counting, and a 0.9 wt.% NaCl solution was used in this process. After the microbial concentration ($C_0$, CFU/ml) at the initial concentration was calculated in consideration of the dilution concentration, an antibacterial activity was derived by calculating the following Mathematical Equation 1.

[Mathematical Equation 1]

$$\text{Antibacterial activity(\%)} = \left(1 - A_{Sample}/A_{Reference}\right) \times 100$$

**$A_{sample}$ = Concentration of microorganisms in medium incubated by adding sample**
**$A_{Reference}$ = Concentration of microorganisms in medium incubated without adding sample**

In the following Table 2, the control is a bacteria inoculation culture solution to which no sample has been added, and is a measure of whether the bacteria have grown properly. In the following Example 4-1 and Comparative Example 4-1, experiments were conducted after confirming the reliability of the experiment through the controls in the following Table 2.

[Table 2]

| | Used sample | CFU/ml | Antibacteri al activity value | Antibacteri al activity (%) |
|---|---|---|---|---|
| Control | - | 1.4E+05 | - | - |
| Example 4-1 | Sample 1 | 3.1E+00 | 4.8 | More than 99.9 |
| Comparative Example 4-1 | Sample 7 | 2.4E+02 | 2.8 | 99.8 |

When the results in Table 2 are combined with those in Table 1, it can be confirmed that samples with low turbidity have high antibacterial activity.

Through this, it can be confirmed that the lower the content of impurities, the lower the turbidity and the better the antibacterial activity.

## Claims

1. A compound comprising a quaternary ammonium structure having an acrylate group or a methacrylate group, wherein a turbidity derived by the following Method 1 is less than 120:

   [Method 1]
   a 20 wt% aqueous solution of the compound is analyzed by UV/Vis spectroscopy to measure the absorbance Abs to light at a wavelength of 290 nm, and a turbidity Tb is derived from the measured absorbance Abs by the following Equation 1.

   [Equation 1]

   $$Abs = 0.00309\ Tb + 0.04946$$

2. The compound of claim 1, wherein the turbidity is less than 40.

3. The compound of claim 1, wherein when the compound is subjected to the following Method 2, no peak of 100 mV or more is detected at 4 minutes to 9 minutes:

   [Method 2]

the compound is dissolved in a mixed solution comprising acetonitrile and water at a volume ratio of 5:5 and analyzed by HPLC/CAD.

**4.** The compound of claim 3, wherein when the compound is subjected to Method 2, a peak is detected at 1 minutes to 3 minutes.

**5.** The compound of claim 1, wherein the compound is subjected to the following Method 3, no peak of 10mV or more is detected at 10 minutes to 18 minutes:

[Method 3]
the compound is dissolved in DMF and analyzed by GPC/RI-DMF.

**6.** The compound of claim 1, wherein the compound is represented by the following Chemical Formula 1:

[Chemical Formula 1]

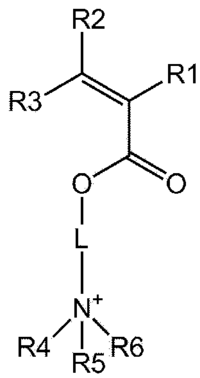

in Chemical Formula 1,
R1 to R3 are the same as or different from each other, and are each independently hydrogen or a methyl group,
R4 to R6 are the same as or different from each other, and are each independently an alkyl group, and
L is an alkylene group.

**7.** The compound of claim 6, wherein any one of R4 to R6 is an alkyl group having 5 to 30 carbon atoms, and the others are the same as or different from each other, and are each independently an alkyl group having 1 to 30 carbon atoms.

**8.** The compound of claim 6, wherein R2 and R3 are hydrogen, R1 is hydrogen or a methyl group, and L is an alkylene group having 1 to 30 carbon atoms.

**9.** The compound of claim 1, wherein the compound is any one selected from the following Compounds 1 to 10:

Compound 1

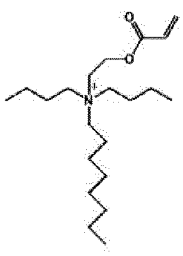

Compound 2

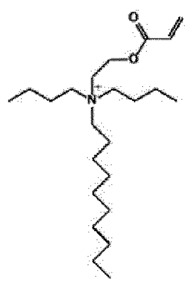

Compound 3

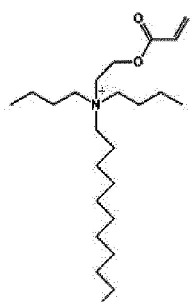

Compound 4

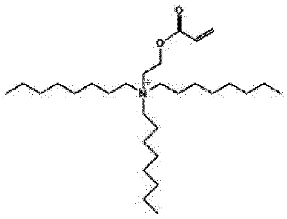

Compound 5

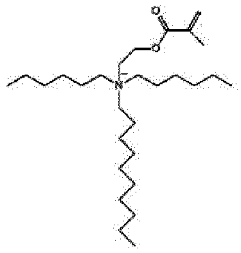

Compound 6

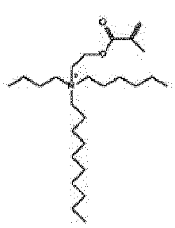

Compound 7

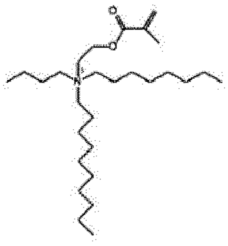

Compound 8

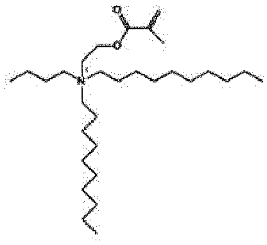

Compound 9

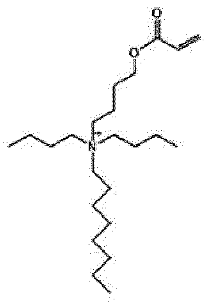

Compound 10

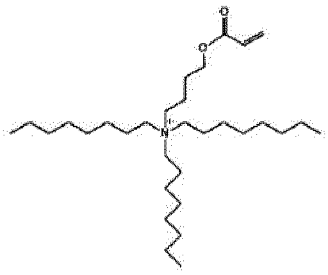

.

**10.** A method for confirming impurities in a compound comprising a quaternary ammonium structure having an acrylate group or a methacrylate group,
wherein the confirmation of impurities is performed by at least one of the following Method 1' to 3':

[Method 1']
a 20 wt% aqueous solution of the compound is analyzed by UV/Vis spectroscopy to measure the absorbance Abs to light at a wavelength of 290 nm, a turbidity Tb is derived from the measured absorbance Abs by the following Equation 1, and when the derived turbidity Tb is 120 or more, the presence of impurities is confirmed.

[Equation 1]

$$Abs = 0.00309\ Tb + 0.04946$$

[Method 2']
the compound is dissolved in a mixed solution including acetonitrile and water at a volume ratio of 5:5 and analyzed by HPLC/CAD, and when a peak of 100 mV or more is detected at 4 minutes to 9 minutes, the presence of impurities is confirmed.
[Method 3']
the compound is dissolved in DMF and analyzed by GPC/RI-DMF, and when a peak of 10 mV or more is

detected at 10 minutes to 18 minutes, the presence of impurities is confirmed.

**11.** An antibacterial composition comprising the compound according to any one of claims 1 to 9.

[Figure 1]

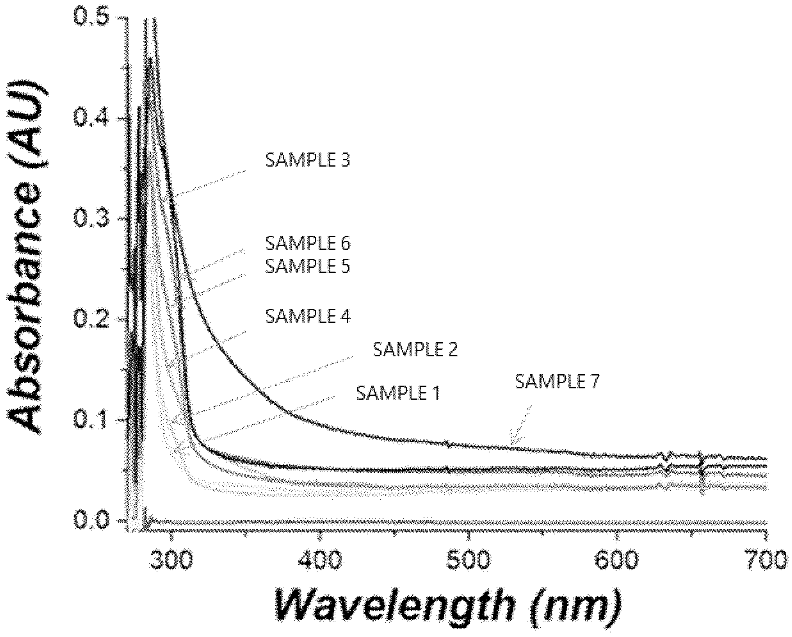


[Figure 2]

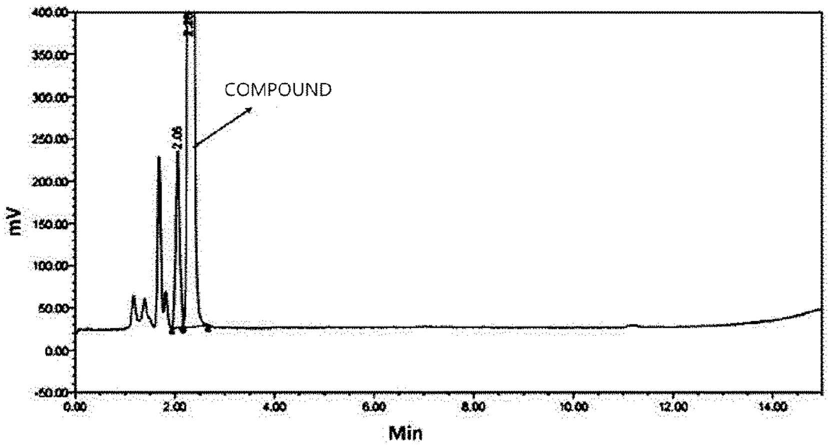

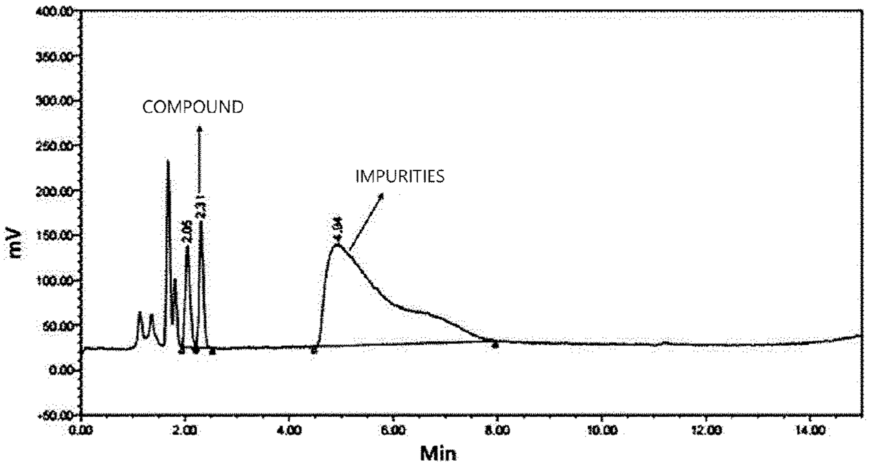


[Figure 4]

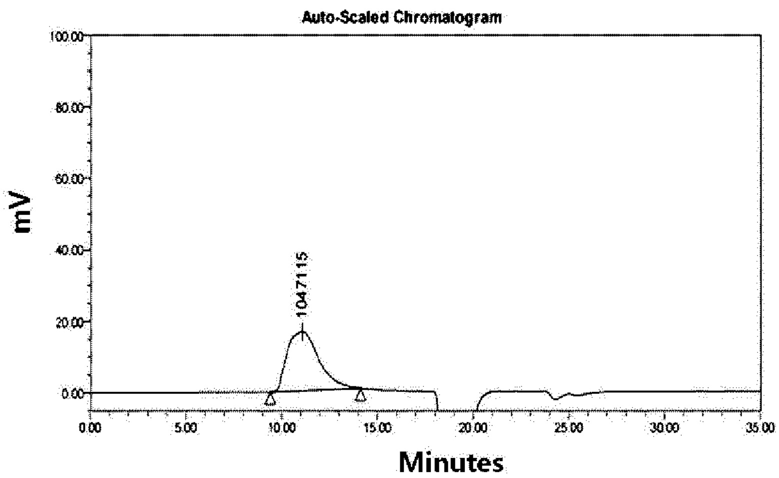


GPC Results

| Mn | Mw | Mp | Poly dispersity |
|---|---|---|---|
| 376458 | 704777 | 1047115 | 1.872124 |

[Figure 5]

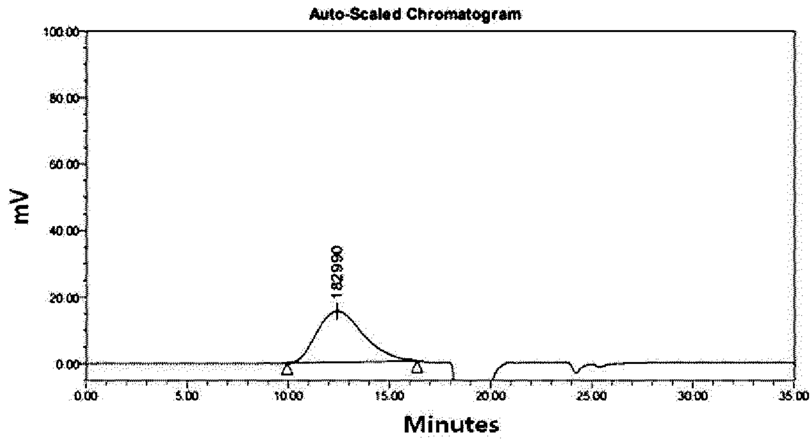


GPC Results

| Mn | Mw | Mp | Poly dispersity |
|---|---|---|---|
| 86899 | 250361 | 182990 | 2.881042 |

[Figure 6]

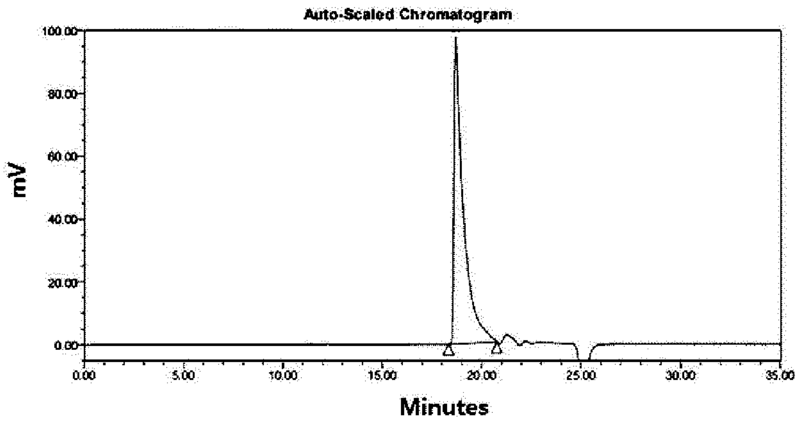


GPC Results

| Mn | Mw | MP | Polydispersity |
|---|---|---|---|
| 370 | 406 | 509 | 1.098346 |

[Figure 7]

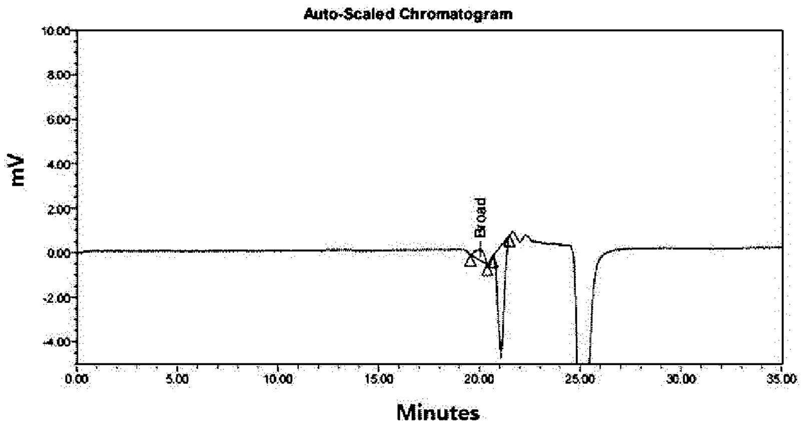


GPC Results

| Mn | Mw | MP | Polydispersity |
|---|---|---|---|
| 173 | 174 | | 1.002725 |

**INTERNATIONAL SEARCH REPORT**

International application No.
**PCT/KR2023/013917**

**A. CLASSIFICATION OF SUBJECT MATTER**

**C07C 219/20**(2006.01)i; **A01N 33/12**(2006.01)i; **G01N 21/31**(2006.01)i

According to International Patent Classification (IPC) or to both national classification and IPC

**B. FIELDS SEARCHED**

Minimum documentation searched (classification system followed by classification symbols)

C07C 219/20(2006.01); A01N 25/12(2006.01); A01N 37/02(2006.01); C07C 229/28(2006.01); C07C 7/00(2006.01); C08F 36/00(2006.01); G01N 30/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus) & keywords: 항균(antimicrobial), 불순물(impurity), 탁도(turbidity), 아크릴레이트기(acrylate), 메타크릴레이트(methacrylate), 4급 암모늄(quaternary ammonium)

**C. DOCUMENTS CONSIDERED TO BE RELEVANT**

| Category* | Citation of document, with indication, where appropriate, of the relevant passages | Relevant to claim No. |
|---|---|---|
| A | FARAH, S. et al. Quaternary ammonium poly(diethylaminoethyl methacrylate) possessing antimicrobial activity. Colloids and surfaces B: biointerfaces. 2015, vol. 128, pp. 608-613.<br>See abstract; formula 1; and figure 2. | 1-11 |
| A | US 2011-0195104 A1 (JIANG, S. et al.) 11 August 2011 (2011-08-11)<br>See entire document. | 1-11 |
| A | US 6629039 B1 (WANG, Y.) 30 September 2003 (2003-09-30)<br>See entire document. | 1-11 |
| A | CN 114402198 A (SICHUAN HAISCO PHARMACEUTICAL CO., LTD.) 26 April 2022 (2022-04-26)<br>See entire document. | 1-11 |
| A | US 2009-0312511 A1 (RAMAGE, D. L. et al.) 17 December 2009 (2009-12-17)<br>See entire document. | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 December 2023** | **29 December 2023** |
| Name and mailing address of the ISA/KR<br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2023/013917**

| Patent document cited in search report | Publication date (day/month/year) | Patent family member(s) | Publication date (day/month/year) |
|---|---|---|---|
| US 2011-0195104 A1 | 11 August 2011 | US 2009-0156460 A1 | 18 June 2009 |
| | | US 2010-0247614 A1 | 30 September 2010 |
| | | US 8268301 B2 | 18 September 2012 |
| | | US 8658192 B2 | 25 February 2014 |
| | | WO 2009-067566 A1 | 28 May 2009 |
| US 6629039 B1 | 30 September 2003 | EP 1167966 A2 | 02 January 2002 |
| | | EP 1167966 A3 | 21 April 2004 |
| | | JP 2002-267648 A | 18 September 2002 |
| | | JP 3532880 B2 | 31 May 2004 |
| | | US 2004-0014146 A1 | 22 January 2004 |
| | | US 6865493 B2 | 08 March 2005 |
| CN 114402198 A | 26 April 2022 | WO 2021-043117 A1 | 11 March 2021 |
| US 2009-0312511 A1 | 17 December 2009 | EP 2043750 A2 | 08 April 2009 |
| | | EP 2043750 B1 | 14 March 2012 |
| | | JP 2009-543930 A | 10 December 2009 |
| | | JP 2013-173947 A | 05 September 2013 |
| | | JP 5378210 B2 | 25 December 2013 |
| | | JP 5774050 B2 | 02 September 2015 |
| | | TW 200811203 A | 01 March 2008 |
| | | US 8013082 B2 | 06 September 2011 |
| | | WO 2008-010962 A2 | 24 January 2008 |
| | | WO 2008-010962 A3 | 24 April 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020220117306